# EUROPEAN PATENT SPECIFICATION

(11) **EP 2 170 244 B1**
(45) Date of publication and mention of the grant of the patent: **30.03.2011**
(21) Application number: 08767316.6
(22) Date of filing: 03.06.2008
(51) Int. Cl.: A61K 6/04

(54) **PROCESS FOR APPLYING ADHESION COATING TO A SUBSTRATE**
VERFAHREN ZUM AUFBRINGEN EINER ADHÄSIONSSCHICHT AUF EINEM SUBSTRAT
PROCÉDÉ D'APPLICATION D'UN REVÊTEMENT D'ADHÉSION SUR UN SUBSTRAT

(30) Priority: 28.06.2007 SI 200700154
(43) Date of publication of application: 07.04.2010
(73) Proprietor: INSTITUT JOZEF STEFAN, 1000 Ljubljana (SI)
(72) Inventor: KOSMAC, Tomaz, 1216 Smlednik (SI); KRNEL, Kristoffer, 1000 Ljubljana (SI); KOCJAN, Andraz, 1000 Ljubljana (SI); JEVNIKAR, Peter, 1000 Ljubljana (SI)
(74) Representative: Liedl, Christine
(86) International application number: PCT/SI2008/000032
(87) International publication number: WO 2009/002278

(56) References cited:
- US-B1- 6 413 660
- RUDDELL, THOMPSON, STONER: "MECHANICAL PROPERTIES OF A DENTAL CERAMIC COATED BY RF MAGNETRON SPUTTERING" J. OF BIOMEDICAL MATERIAL RES., vol. 51, no. 3, 22 June 2000 (2000-06-22), - 22 June 2000 (2000-06-22) pages 316-320, XP002554265
- BOWEN, HIGHFIELD, MOCELLIN, RING: "DEGRADATION OF ALUMINIUM NITRIDE POWDER IN AN AQUEOUS ENVIRONMENT" J. AM. CERAM. SOC., vol. 73, no. 3, 1 March 1990 (1990-03-01), - 30 March 1990 (1990-03-30) pages 724-728, XP002554118
- FREEMAN, BROOK: "BONE RESPONSE TO A TITANIUM ALUMINIUM NITRIDE COATING ON METALLIC IMPLANTS" J. MATER. SCI. : MATER. MED., vol. 17, no. 5, 1 May 2006 (2006-05-01), - 31 May 2006 (2006-05-31) pages 465-470, XP002554119

## Description

The present invention relates to a process for applying an adhesion coating to a substrate, preferably a fixed partial denture (FPD) or a bone implant, using precipitation of aluminum hydroxides which originate from the hydrolysis of aluminum nitride - AlN - powder in a water suspension, or a water-containing suspension, followed by a subsequent thermal treatment. The purpose of the coating is to improve adhesion of commercially available dental and/or bone cements to the substrate, which is preferably used as a fixed partial denture or a bone implant.

Sintered oxide ceramics, particularly tetragonal zirconium oxide ceramics, is being increasingly employed in recent times as a material for fashioning fixed partial dentures, such as inlays, crowns, bridges, posts and implant abutments, and bone implants in orthopedics. Such ceramic materials are chemically inert both in the oral cavity and in contact with the biological tissues of the body, they are non-toxic and bio-compatible, don't irritate the mucous membranes, don't cause inflammations or allergies and don't color the gums. Besides possessing adequate mechanical properties, allowing protracted clinical use, the said materials also exhibit good optical properties which confer an aesthetic appearance to the fixed partial denture.

One of the disadvantages of sintered oxide ceramics, particularly for dental applications, is poor adherence to the dental cement, wherewith it is fixed to the supporting teeth. In the aggressive environment of the oral cavity, the ceramic-cement-dentin system is exposed to thermal, chemical and mechanical stresses which may weaken the bonding strength and the stability of the union. There are two factors affecting the bond, i.e. the adhesion: the mechanical factor is based on the surface topography and may be described as surface energy; the larger the surface, the higher the surface energy and consequently the better the adhesion. The other factor is the chemical bond which depends on the chemical interaction between the adhesive and the adherent. If there is no such interaction, only weak Van der Waals forces contribute to the bonding strength, the said forces generally not providing sufficient strength to the union. Commercially available dental cements exhibit a good bonding to hard dental tissues, since both the said moments contribute to the union strength; they however bond poorly to the surface of the chemically inert sintered dental ceramics, in particular tetragonal zirconium oxide ceramics and aluminum oxide ceramics. Ceramic materials of this type are also resistant to acids, so that the surface can not be etched with simple techniques, as opposed to, say, dental ceramics that contains silicates. Therefore, for improving the adhesion of dental cements to sintered ceramic fixed partial dentures, alternative methods must be employed, aimed at either increasing the mechanical retention, or increasing the chemical interaction between the adhesive and the adherent.

Several methods for improving the bonding of sintered ceramics to dental cements are known in the art. The simplest method consists in sandblasting the surface of the ceramic fixed partial denture with corundum (Al₂O₃), wherewith the roughness of the surface is increased, resulting in better mechanical bonding (Kern M, Wegner SM. Bonding to zirconia ceramic: adhesion methods and their reliability. Dent Mater 1998; 14: 64-71; Wolfart M, Lehman F, Wolfart S, Kern M. Durability of the resin bond strength to zirconia ceramic after using different surface conditioning methods. Dent Mater 2007; 23: 45-50). Opinions are divided on whether it is appropriate to subject sintered dental ceramics to sandblasting, given that sandblasting may induce surface cracks, which may subsequently, under long-term clinical stresses, extend in depth, thereby reducing the strength of the fixed partial denture. For example, Zhang et al. (Zhang Y, Lawn BR, Rekow ED, Thompson VP: Effect of sandblasting on the long term performance of dental ceramics. J Mater Res Part B: Appl Biomater 2004; 71B: 381-6) report that sandblasting zirconium oxide ceramics having a high initial strength in the order of 2400 MPa and a lower toughness reduces strength for both static and cyclic loads. On the other hand, sandblasting coarse-grained ZrO₂ ceramics having a lower strength and a higher fracture toughness improves the mechanical properties and the reliability of zirconium oxide ceramics (Kosma 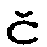 T, Oblak 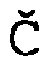 , Jevnikar P, Funduk N, Marion L: Strength and Reliability of Surface Treated Y-TZP Dental Ceramics. J Biomed Mater Res 2000; 53: 304-13). Owing to the higher transformability of the coarse-grained tetragonal zirconium oxide ceramics, part of the tetragonal grains are transformed into the monoclinic form when subjected to sandblasting, as a result of which compression stresses arise on the surface of the ceramics. These compression stresses partially compensate external tensile stresses, thus potentially improving the mechanical characteristics (Garvie RC, Hannink RH, Pascoe RT., Ceramic Steel? Nature 1975; 258: 703-4; Green DJ., A technique for introducing surface compression into zirconia ceramics, J Am Ceram Soc 1983; 66: C178-C9). The phase transformation of ZrO₂ from the tetragonal into the monoclinic form (t→m) being reversible, the positive impact of sandblasting on the mechanical properties of coarse-grained tetragonal zirconium oxide ceramics is lost if the ceramics is subsequently heated above the temperature of the reverse (m→t) transformation, as in veneering porcelain.

Likewise, several chemical techniques are known for depositing silicon oxide (SiO₂) on the surface of the substrate, all based on the synthesis of SiO₂ from solutions of organo-silicon complexes, i.e. silanes. One of such techniques is set forth in US 6413660.

Another method known in the art is that of tribochemical deposition of silicon oxide (SiO₂) on a substrate with sandblasting, using corundum grains modified, i.e. coated, with SiO₂. This method, developed already in the Eighties of the 20^{th} century, primarily to improve bonding of plastic materials to metal surfaces in the faceting technique (Peutzfeldt A, Asmussen E, Silicoating: evaluation of a new method of bonding composite resin to metal. Scand J Dent Res 1988; 96: 171-6), was later utilized also to improve bonding of zirconium oxide ceramics to dental cement. Several systems are currently in use, the most diffused thereof being the Rocatec system manufactured by 3M ESPE, its derivative CoJet being of clinical use as well. In both the said systems the tribochemical SiO₂ coating on the ZrO₂ surface is combined by subsequent silanization, i.e. synthesis of SiO₂ from solutions of organo-silicon complexes. Although both techniques of depositing SiO₂ on the surface of the substrate are being used in combination (Matinlinna JP, Heikkinen T, Ozcan M, Lassila LVJ, Vallittu P. Evaluation of resin adhesion to zirconia ceramic using some organosilanes. Dent Mater 2006; 22: 824-31), it has been determined that the creation of polysiloxane bonds is confined to localized surface areas. In fact, by analyzing the elemental composition of the surface, only 11% of SiO₂ presence was ascertained after treating the surface with the Rocatec treatment. On the other hand, pre-clinical tests indicate that, as a preparation technique, silanization combined with tribochemical surface treatment of the Rocatec system is more effective than sandblasting with unmodified, i.e. non-coated corundum grains (Luthy H, Loeffel O, Hammerle CHF. Effect of thermocycling on bond strength of luting cements to zirconia ceramics. Dent Mater 2006; 22: 195-200; Bitter K, Priehn K, Martus P, Kielbassa A. In vitro evaluation of push-out bond strengths of various luting agents to tooth colored posts. J Prosthet Dent 2006; 95: 302-10). However, given that in the Rocatec system the target is being bombarded by particles under a higher pressure and with a higher velocity as opposed to the commonly used technique (Kern M, Thompson VP. Effects of sandblasting and silica-coating procedures on pure titanium. J Dent 1994; 22: 300-6), there is also an increased risk of surface cracks being formed, which in turn may prevail over the positive effect of the transformation-induced surface hardening. In addition, according to the assessments of Kern et al. (Kern M, Wegner SM. Bonding to zirconia ceramic: adhesion methods and their reliability. Dent Mater 1998; 14: 64-71), the long-term clinical effectiveness of the described tribochemical procedures is questionable due to the instability of the polysiloxane bond in a wet environment.

In addition to the said techniques which are more or less employed in practice, a process is also known in the art for depositing a ceramic adhesive layer on a substrate by dip-coating, followed by subsequent thermal treatment. According to the said process, particles of different ceramic materials may be deposited on the substrate, the disadvantage of the process being in that the coatings obtained by dip-coating are relatively thick, from several tens of micrometers to several hundreds of micrometers, as a result whereof they crack when subjected to subsequent thermal treatment and are therefore not fit for practical use. The said disadvantage is partially overcome by employing the electrophoretic deposition process, which is however not used in current practice due to geometric limitations and difficult execution, resulting in high final costs.

Additionally, techniques are known in the art for applying a chemical covering to a substrate by employing plasma, for example from a vapor phase (PEMOCVD), as set forth in US 6113993. Said techniques are used in practice for depositing bioactive, e.g. hydroxyapatite, coatings onto metal bone implants, although they could also be employed for adhesion coatings on a ceramic substrate.

Still further, a metallization process is known, as described for example in the published patent application WO 01/34097. The process, first developed for galvanic coatings of precious metals on non-noble dental alloys, was subsequently modified and utilized for strengthening mechanically weak dental porcelains and glass ceramics. In addition to improving the mechanical strength, the said metallic coating may also improve bonding to dental cements, although the process is costly and the galvanic covering impairs the aesthetic appearance of the fixed partial denture, diminishing the comparative advantage of ceramics as compared to metals.

The technical problem that, according to available data, has not yet been adequately solved is a long-term and clinically effective improvement in the adhesion of dental cements to the substrate, which is preferably, but not exclusively, made of sintered oxide ceramics and is preferably meant to be used in dental prosthetics.

It is the object of the present invention to set forth a simple process for fabricating an adhesion coating, i.e. a layer deposited on a substrate, preferably a fixed partial denture or a bone implant made of sintered oxide ceramics, which will have a large surface, thus improving the mechanical adhesion to common tooth or bone cements, will be thin enough not to crack when subjected to subsequent thermal processes, and will locally form strong chemical bonds with common dental or bone cements, whereby the adhesion of the substrate to natural hard tissue, such as dentin, will be improved over the methods known in the art.

In accordance with the invention, the object is achieved with synthesizing a thin porous adhesion coating to a substrate as set forth in the independent patent claims.

For fabricating the adhesion coating, i.e. applying the covering layer to the substrate as per the invention, sintered ceramics from tetragonal zirconium oxide, aluminum oxide, spinel, mullite or any other biocompatible inorganic material, fit for use as dental material or bone implant material is employed as substrate. The substrate is immersed for at least 0.1 min, preferably for 5 to 60 min, in water or another water-containing liquid medium, having a pH of 1 to 14, preferably from 5 to 11, and additionally containing aluminum nitride (AlN) powder with the average particle size of 5 nanometers to 100 micrometers, preferably from 0.2 micrometers to 15 micrometers, wherein the mass percentage of the AlN powder in water or water-containing medium is from 1 % w/w to 70 % w/w, preferably from 2 % w/w to 15 % w/w. The synthesis of the covering layer, i.e. coating, takes place with the precipitation of aluminum hydroxides which originate from the hydrolysis of AlN powder in water suspension, or in water-containing suspension, preferably at atmospheric pressure and at a temperature between the freezing point and the boiling point of water, preferably in the region between 40°C and 95°C; after soaking, the substrate is taken out of the suspension, dried and subjected to thermal treatment, namely heating, at temperatures from 400°C to 1350°C, preferably at temperatures from 600°C to 1100°C. The thermal treatment according to the invention may be carried out as an independent operation, or it can be carried out together with the thermal treatment of porcelains and/or glazes and/or other additional coatings applied to the substrate.

The invention shall hereinafter be described in more detail with reference to the preferred embodiments thereof.

First embodiment. As substrate, a densely sintered disc with a diameter of 15 mm and a height of 2 mm, made of tetragonal ZrO₂, stabilized with 3 % mol Y₂O₃ was used, sandblasted on one side with 110-micron corundum grains. The distance between the substrate and the sandblasting nozzle with a diameter of 5 mm was 30 mm, and the sandblasting period lasted 15 s at the pressure of 4 bars. The substrate was then dipped in a suspension, prepared by dispersing 20 g of AlN powder with 3 micrometer average particle size in 250 ml of distilled water having a pH value of 6 and a temperature of 50°C. After mixing the AlN powder into the distilled water, the pH value of the suspension increased to 10.5. After 60 min the substrate was taken out of the suspension and subsequently dried in a drier for 2 hours at 110°C. After drying, the substrate was thermally treated by being heated in an electric resistance oven in atmospheric air at a temperature of 600°C. The heating at the said temperature lasted 2 hours. Adhesion of the dental cements to the substrate was tested with a shear test according to the ISO 6872:1995(E) standard, wherein two different cements were used, composite cement and glass-ionomer cement. The movable blade mounted in the measuring probe was traveling at the speed of 1 mm/min. The samples were subjected to loads until breaking down. The breakpoint force was registered and the shear bond strength was calculated, which is the measure of the adhesion power of the cement to the substrate. The values for the substrate with the adhesion coating were compared to the bonding strength on sandblasted substrates having no adhesion coating. The shear bond strengths on the reference substrates with the composite cement and the glass ionomer cement were 8.6 ± 1.5 MPa and 7.3 ± 2 MPa, respectively, whereas the shear bond strengths on the substrates having the inventive coating were 22.4 ± 4 MPa and 20.3 ± 3.5 MPa, respectively.

Second embodiment. As substrate, a densely sintered corundum (Al₂O₃) disc with a diameter of 15 mm and a height of 2 mm was used, ground on one side with diamond pastes and polished to mirror surface. The substrate was immersed in 100 ml of 0.1 M solution of magnesium acetate with a pH value of 3.4. The solution was heated to 95°C and 1.5 g of AlN powder was added with an average particle size of 0.3 micrometers. After 2 min the substrate was taken out of the AlN powder suspension in magnesium acetate solution and dried for 2 hours in a drier at 110°C. After drying, the substrate was heated to 1000°C in atmospheric air in an electric resistance oven. The heating at the said temperature lasted 15 min. The shear bond strength of the glass ionomer cement on the reference substrate having no coating was 8.1 ± 1.7 MPa, while the shear bond strength on the substrates with the inventive coating was 19.8 ± 3 MPa.

Third embodiment. As substrate, a cylindro-conical dental post with a support of densely sintered tetragonal ZrO₂ ceramics, stabilized with 3 mol % Y₂O₃ was used. The post having a cylindrical portion of 1.7 mm in diameter and a conical portion of 8 mm in length was immersed in a suspension, prepared by dispersing 60 g of AlN powder with an average particle size of 3 micrometers in 250 ml of 25 % solution of ethylene glycol in distilled water. The suspension with the substrate immersed therein was heated to 30°C. After 6 hours the substrate was taken out of the suspension and dried in a drier at 110°C. After drying, the substrate was heated to 1000°C in atmospheric air in an electric resistance oven. The heating at the said temperature lasted 15 min. For measuring the adhesion of the cement to the substrate, artificial root canals were fabricated in methylmetacrylate (Tehnovitu 4071, Heraus, Germany), wherein the posts were then inserted and cemented with composite cement. After the cement had hardened, the conical portions of the posts inside the root canals were sawed into 2 mm thick slices. The ceramic portion was then subjected to load in its narrow part by means of a steel pin 1 mm in diameter and the required push-out force was measured.

To calculate the bonding area, the equation A=π(R₁+R_{2)√}(R₁-R₂₎²+h² was used, wherein R₁ and R₂ are the upper and lower radius of the post in the slice, and h is the thickness of the slice. The push-out bond strength for the reference post was 14.5 ± 3.6 MPa, whereas for the posts with the inventive coating it was 32 ± 4.2 MPa.

## Claims

1. A process for applying adhesion coating to a substrate, preferably a fixed partial denture, the purpose of the coating being to improve the adhesion of commercially available dental and/or bone cements to the substrate, **characterized in that** sintered ceramics from tetragonal zirconium oxide, aluminum oxide, spinel, mullite, or any other bio-compatible inorganic material fit for use as dental material or bone implant material is used as substrate, and the substrate is immersed for at least 0.1 min, preferably for 5 to 60 min in water or a water-containing liquid medium having a pH value of 1 to 14, preferably from 5 to 11, and containing aluminum nitride (AlN) powder with the average particle size from 5 nanometers to 100 micrometers, preferably from 0.2 micrometers to 15 micrometers, the mass portion of AlN powder in water or water-containing medium being from 1 % w/w to 70 % w/w, preferably from 2 % w/w to 15 % w/w, wherein the synthesis is carried out with the precipitation of aluminum hydroxides which originate from the hydrolysis of AlN powder in water suspension, or in water-containing suspension, preferably at atmospheric pressure and at a temperature between the freezing point and the boiling point of water, preferably in the region between 40°C and 95°C, whereupon the substrate is taken out of the suspension, dried and subjected to thermal treatment at temperatures from 400°C to 1350°C, preferably at temperatures from 600°C to 1100°C.

## Patentansprüche

1. Verfahren zum Aufbringen einer Haftbeschichtung auf ein Substrat, vorzugsweise eine feste Teilzahnprothese, wobei die Beschichtung zum Zwecke der Verbesserung der Haftwirkung der im Handel erhältlichen Dental- und/oder Knochenzemente auf dem Substrat vorgesehen ist, **dadurch gekennzeichnet, dass**
Sinterkeramik aus tetragonalem Zirkondioxid, Aluminiumoxid, Spinell, Mullit, oder irgendeinem anderen biologisch kompatiblen anorganischen Material, das für die Verwendung als Dentalmaterial oder Knochenimplantatmaterial geeignet ist, als Substrat verwendet wird, und das Substrat für wenigstens 0,1 min, vorzugsweise für 5 bis 60 min in Wasser oder Wasser enthaltendes flüssiges Medium mit einem pH-Wert von 1 bis 14, vorzugsweise von 5 bis 11, getaucht wird, und Aluminiumnitrid(AlN)-Pulver mit einer durchschnittlichen Teilchengröße von 5 nm bis 100 µm, vorzugsweise von 0,2 µm bis 15 µm, enthält, wobei der Massenanteil von AlN-Pulver in Wasser oder in dem Wasser enthaltenden Medium von 1% (w/w) bis 70 % (w/w), vorzugsweise von 2 % (w/w) bis 15 % (w/w) beträgt, wobei die Synthese unter Ausscheidung von Aluminiumhydroxid, das von der Hydrolyse von AlN-Pulver in wässriger Lösung oder in einer Wasser enthaltenden Suspension stammt, vorzugsweise bei Umgebungsdruck und bei einer Temperatur zwischen dem Gefrierpunkt und dem Siedepunkt von Wasser, vorzugsweise in dem Bereich von zwischen 40°C und 95°C erfolgt, woraufhin das Substrat aus der Suspension genommen, getrocknet und einer Wärmebehandlung bei Temperaturen von 400°C bis 1350°C, vorzugsweise bei Temperaturen von 600°C bis 1100°C, unterzogen wird.

## Revendications

1. Procédé d'application d'une couche d'adhérence à un support, de préférence à une prothèse partielle fixe, l'objectif de la couche étant d'améliorer l'adhérence sur le support de ciments dentaires et/ou osseux disponibles dans le commerce, **caractérisé en ce que** des céramiques frittées d'oxyde de zirconium tétragonal, d'oxyde d'aluminium, de spinelle, de mullite ou de n'importe quel autre matériau inorganique bio-compatible approprié à une utilisation comme produit dentaire ou produit d'implantation osseuse sont utilisés comme support, et **en ce que** le support est immergé pendant au moins 0,1 minute, de préférence pendant 5 à 60 minutes dans de l'eau ou dans un milieu liquide contenant de l'eau qui présente une valeur pH allant de 1 à 14, de préférence de 5 à 11 et contenant une poudre de nitrure d'aluminium (AlN) avec une grosseur de particule moyenne allant de 5 nanomètres à 100 micromètres, de préférence de 0,2 micromètres à 15 micromètres, la portion de masse de poudre AlN dans l'eau ou dans le milieu contenant de l'eau allant de 1% m/m à 70% m/m, de préférence de 2% m/m à 15% m/m, dans lequel la synthèse est effectuée par précipitation d'hydroxydes d'aluminium provenant de l'hydrolyse de la poudre d'AlN dans une suspension d'eau ou dans une suspension contenant de l'eau, de préférence à pression atmosphérique et à une température comprise entre le point de congélation et le point d'ébullition de l'eau, de préférence dans une plage entre 40°C et 95°C, après quoi le support est enlevé de la suspension, séché et exposé à un traitement thermique à des températures allant de 400°C à 1350°C, de préférence à des températures allant de 600°C à 1100°C.
